# EUROPÄISCHE PATENTSCHRIFT

(11) **EP 4 149 570 B1**
(45) Veröffentlichungstag und Bekanntmachung des Hinweises auf die Patenterteilung: **20.08.2025**
(21) Anmeldenummer: 21725083.6
(22) Anmeldetag: 06.05.2021
(51) Int. Cl.: A61L 2/20, A61L 2/22, B05B 1/20, B05B 17/06, A61L 2/00, A61B 90/80, A61H 35/00

(54) **DESINFEKTIONSVORRICHTUNG**
DISINFECTION DEVICE
DISPOSITIF DE DÉSINFECTION

(30) Priorität: 12.05.2020 DE 102020112847
(43) Veröffentlichungstag der Anmeldung: 22.03.2023
(73) Patentinhaber: Krömker Holding GmbH, 31675 Bückeburg (DE)
(72) Erfinder: KRÖMKER, Wilfried, 31675 Bückeburg (DE)
(74) Vertreter: Kröncke, Rolf
(86) Internationale Anmeldenummer: PCT/EP2021/062011
(87) Internationale Veröffentlichungsnummer: WO 2021/228682

(56) Entgegenhaltungen:
- WO-A1-2012/013539
- CN-B- 105 688 239
- FR-A1- 2 617 716

## Beschreibung

Die Erfindung betrifft eine Desinfektionsvorrichtung zur Desinfektion von Oberflächen mit einem Ozongenerator zur Erzeugung eines ozonhaltigen Luftstroms, einem Aerosolgenerator zur Erzeugung eines wässrige Partikel enthaltenen Aerosolstroms und einer Desinfektionskammer, die eine Einlassöffnung zur Einführung eines Objektes mit der zu desinfizierenden Oberfläche in die Desinfektionskammer hat, wobei der Ausgang des Aerosolgenerators mit dem Ausgang des Ozongenerators gekoppelt ist und der von dem Ozongenerator erzeugte ozonhaltige Luftstrom mit dem Aerosolstrom des Aerosolgenerators vermischt und in die Desinfektionskammer geleitet wird.

Zur Desinfektion von Oberflächen, insbesondere zur Handdesinfektion in medizinischen Einrichtungen werden Desinfektionslösungen verwendet, die z. B. auf die zu desinfizierenden Hände aufgetragen werden.

EP 2 223 704 A1 beschreibt eine Einrichtung zur Desinfektion z. B. von Händen mit einem nicht-thermischen Plasma, das in ein einseitig offenes Gehäuse einströmt.

US 6,706,243 B1 offenbart eine Vorrichtung zur Handreinigung, bei der optional ein Plasmagasstrom in einen Handreinigungsraum geleitet werden kann. Zusätzlich werden die Hände mit einem durch eine Ionenquelle geleiteten Luftstrom unter Druck gereinigt. Eine Reinigungslösung wird in einen Gasstrom gepumpt. Der mit Reinigungslösung vermischte Gasstrom wird vernebelt und in den Plasmagenerator geleitet.

US 2013/0272929 A1 beschreibt eine Desinfektionsvorrichtung mit einer Fluidquelle und einem Plasmagenerator zur Erzeugung nicht-thermischen Plasmas. Das Fluid wird mit Plasma aktiviert indem das plasmaaktivierte Fluid aus einer Düse in dem Plasmaerzeugungsraum zwischen Elektroden geleitet wird.

WO 2014/135254 A1 offenbart ein Verfahren und eine Vorrichtung zur Reinigung eines Gegenstands für Desinfektionszwecke, bei dem ein Trägergas und ein Nebel einer Behandlungsflüssigkeit erzeugt und durch einen Plasmagenerator geleitet wird. Dabei wird eine Wechselwirkung der vernebelten Behandlungsflüssigkeit mit dem Plasma zur Herstellung aktivierten Nebels genutzt.

WO 00/67805 A1 offenbart eine Vorrichtung zur Handreinigung, bei der eine Reinigungslösung mit einem Gasstrom vermischt und durch einen Plasmagenerator geleitet wird. Die Handdesinfektion erfolgt zweistufig zunächst mit einem nicht mit Aerosolen versetzten trockenen Plasmagasstrom und anschließend mit Hilfe der im Hochspannungsfeld aktivierten Reinigungslösung.

EP 3 041 518 B1 offenbart eine gattungsgemäße Desinfektionsvorrichtung zur Plasmadesinfektion von Oberflächen mit einem Plasmagenerator zur Erzeugung eines desinfizierenden Plasmagasstroms und mit einem mit dem Plasmagenerator in Verbindung stehenden, mindestens teilweise geschlossenen Desinfektionsbereich. Weiterhin ist ein Aerosolgenerator zur Erzeugung eines wässrige Partikel enthaltenen Aerosolstroms vorgesehen, der mit dem Plasmagenerator in Verbindung steht, um einen mit dem Aerosolstrom vermischten Plasmagasstrom in dem Desinfektionsbereich auf die zu desinfizierende Oberfläche zu leiten. Der Aerosolgenerator ist an den Plasmagasstrom-Ausgang des Plasmagenerators gekoppelt, wobei der vom Plasmagenerator erzeugte Plasmagasstrom mit dem Aerosolstrom des Aerosolgenerators vermischt und in den Desinfektionsbereich geleitet wird.

Dadurch werden die Aerosole nicht durch den Plasmagenerator selbst beeinträchtigt. Es wird vermieden, dass es im Plasmagenerator durch das Wasser-Luft-Gemisch zu einem Quenching des erzeugten Plasmas kommt, d. h. dass die Intensität des Plasmas durch wässrige Partikel wesentlich reduziert wird bzw. das Plasma partiell gelöscht wird und damit an Wirkung verliert. Der Wasseranteil H₂O reagiert mit dem Ozon O₃ und durch die Reaktionsprodukte wird die Desinfektionswirkung des Aerosolnebels signifikant verbessert.

Wenn der Plasmagasstrom durch das Volumen des Aerosols geleitet wird, kann eine hinreichende Verweildauer sichergestellt werden, die eine Modifizierung bzw. Reaktion der Aerosole mittels des Plasmagasstroms gewährleistet.

FR 2 617 716 A1 beschreibt ein Gerät zum Sterilisieren von Händen und Unterarmen, das eine äußere Hülle umfasst, die ein Gehäuse bildet und eine Kammer begrenzt, sowie mobile Mittel zum Versprühen einer Desinfektionsflüssigkeit. Diese Mittel sind im Inneren der Kammer angeordnet und werden aus einem Tank mit Desinfektionsflüssigkeit versorgt und durch das Einführen der Hände in die Kammer aktiviert. Zur Verbesserung der Wirkung der Sterilisationsflüssigkeit hat das Gerät außerdem einen Ozonisator, der Luft von außen ansaugt und einen Teil des in der Luft enthaltenen Sauerstoffs in Ozon umwandelt. Dieser ozonisierte Luftstrom wird parallel und unabhängig von der eingedüsten Desinfektionsflüssigkeit in die Kammer eingeblasen.

CN 10588239 B offenbart eine antibakterielle Behandlungsvorrichtung mit Ultraschallzerstäubung, bei der eine antibakterielle Flüssigkeit in einen Mischflüssigkeitstank einer Zerstäubungskammer gegossen, durch einen im Mischflüssigkeitstank angeordneten Ultraschallzerstäuber zu einer Sprühzubereitung zerstäubt und in der Zerstäubungskammer eine Konzentrationsakkumulation durchgeführt wird. Die erste Öffnung eines Behandlungsraums kommuniziert mit der Zerstäubungskammer. Das Sprühpräparat wird in den Behandlungsraum überführt. Ein zu behandelnder Gegenstand wird in dem Behandlungsraum angeordnet und durch Drehen der Vorrichtung in Rotation versetzt. Das Sprühpräparat wird gleichmäßig auf der Oberfläche des zu behandelnden Objekts verteilt und die antibakterielle Behandlung damit vorgenommen. Das antibakterielle Behandlungsgerät mit Ultraschallzerstäubung ist mit einem Ozongenerator ausgestattet, der separat von der Ultraschallzerstäubung kommunizierend mit dem Behandlungsraum verbunden oder in dem Behandlungsraum angeordnet ist, um den Sterilisationseffekt zu erhöhen.

WO 2012/013539 A1 zeigt ein Hausgerät mit einem Ozongenerator und einem Nebelgenerator, die beide an einen Mischer angeschlossen sind. Der Ausgang des Mischers ist mit dem Behandlungsbereich verbunden, um ozonhaltigen Nebel in den Behandlungsbereich zu führen. Der Mischer ist bevorzugt als Venturirohr ausgebildet.

Der Plasmagasstrom ist durch desinfizierendes Ozon, das durch seine freie Radikale sehr instabil ist, und deren Reaktionsprodukte charakterisiert.

Ein Problem ist es, eine insbesondere für den Einsatz in medizinischen Bereichen reichende Desinfektionswirkung sicherzustellen, d. h. eine ausreichend große Log-Stufe von bevorzugt Log4 und mehr. Höhere Abtötungsraten wie Log5 und mehr sind vorteilhaft.

Die Aufgabe der vorliegenden Erfindung ist es daher, eine verbesserte Desinfektionsvorrichtung zu schaffen, die zuverlässig und schnell eine hohe Desinfektionswirkung auf den Oberflächen entfaltet.

Die Aufgabe wird mit der Desinfektionsvorrichtung mit den Merkmalen des Anspruchs 1 gelöst. Vorteilhafte Ausführungsformen sind in den Unteransprüchen beschrieben.

Es wird davon ausgegangen, dass zusätzlich zu dem Aerosolgenerator noch eine hiervon räumlich getrennte Mischkammer vorgesehen ist, wobei der Aerosolgenerator und der Ozongenerator mit der Mischkammer zum Einleiten des Aerosolstroms und des ozonhaltigen Luftstroms in die Mischkammer verbunden sind. Die Desinfektionskammer hat Eindüsungsöffnungen, die mit der Mischkammer verbunden sind, um den mit dem ozonhaltigen Luftstrom vermischten Aerosolstrom als Desinfektionsmedium in die Desinfektionskammer zur Desinfektion der Oberflächen eines in der Desinfektionskammer eingeführten Objektes einzuleiten.

Die Vermischung des Aerosolstroms mit dem ozonhaltigen Luftstrom erfolgt somit in einer separaten Mischkammer, die von dem Plasmagenerator, dem Aerosolgenerator und der Desinfektionskammer getrennt ist. Diese Mischkammer ist nicht Teil der Desinfektionskammer. Vielmehr wird der aerosolhaltige Ozonstrom aus der Mischkammer über Eindüsungsöffnungen in die Desinfektionskammer geleitet.

Die Mischkammer ist erfindungsgemäß ein Paraboloid.

Durch diese separate Mischkammer in Form eines Paraboloids wird erreicht, dass der ozonhaltige Luftstrom nicht durch die im Aerosolgenerator noch vorherrschende Energie des Aerosolstroms beeinträchtigt wird. In der Mischkammer wird zudem eine hinreichende Vermischung vorzugsweise durch Verwirbelung von Aerosolstrom und ozonhaltigem Luftstrom erreicht.

Durch diese separate Mischkammer als Paraboloid gelingt es, den Wirkungsgrad der Desinfektionsvorrichtung signifikant zu erhöhen und reproduzierbar zu gestalten. Es hat sich gezeigt, dass die in der Mischkammer ionisierten Aerosole maßgeblich zur Desinfektion beitragen.

Wenn der Aerosolstrom als Hauptstrom für die Desinfektion genutzt wird, indem der ozonhaltige Luftstrom in den bereits in die Mischkammer eingeströmten Aerosolstrom eingemischt wird, werden die Aerosole ionisiert. Diese dadurch erheblich desinfizierend wirkenden Aerosole sind an sich sehr instabil. Dadurch, dass sie zusammen mit dem verbleibenden ozonhaltigen Luftstrom zur Desinfektionskammer geführt werden, bleibt ihre Ionisierung und damit ihre desinfizierende Wirkung erhalten. Durch die Bereitstellung eines angepassten Volumens an Aerosolstrom und ozonhaltigem Luftstrom kann die Keimreduktionsrate verbessert werden. Es wurde erkannt, dass die ionisierten Aerosole eine größere desinfizierende Wirkung haben, als ein ozonhaltiger Luftstrom. Dieser ozonhaltige Luftstrom ist allerdings auch für die Desinfizierung in der Desinfektionskammer hilfreich, insbesondere aber zum störungsfreien Transport der Aerosole zur Desinfektionskammer erforderlich.

Für den vorbeschriebenen physikalisch-chemischen Vorgang sollten die Mengen zueinander passend gewählt werden. Dies kann einfach durch Versuche anhand der jeweiligen Konstruktion optimiert werden. Die Pumpmenge durch den Ozongenerator sollte dabei an das Volumen des Handraums angepasst werden. So ist vorteilhaft, wenn bei einem Handraumvolumen von drei Litern die Pumpmenge durch den OzonGenerator in einer Einschaltzeit von 30sec. etwas kleiner als das Handraumvolumen ist, um einen ausreichenden Zustrom des Aerosols zu ermöglichen, da der Druck im Aerosolstrom geringer als im Ozonstrom ist.

Der Aerosolgenerator kann eingerichtet sein, um den Aerosolstrom durch Vernebelung mittels Ultraschall von destilliertem Wasser zu erzeugen. Durch die Nutzung von destilliertem Wasser wird eine Reinheit des Fluides sichergestellt, bei der ein Quenchen des ozonhaltigen Luftstroms durch störende Fremdkörper oder chemische Bestandteile sicher vermieden wird. Durch die Vernebelung mittels Ultraschall wird ein sehr feiner Aerosolnebel bereitgestellt. Dieser hat allerdings den Nachteil, dass durch den Ultraschallaktuator eine hohe Energie in die Aerosole eingebracht wird, was sich nachteilig auf den ozonhaltigen Luftstrom bei der Vermischung auswirkt. Durch den Transportweg vom Ozongenerator zur Mischkammer wird dieser nachteilige Effekt reduziert.

Der Aerosolgenerator kann auch eingerichtet sein, um den Aerosolstrom durch Eindüsen von destilliertem Wasser mittels Düsen zu erzeugen. Hierzu wird ein Wasserdruck aufgebaut, der das Fluid durch die Düsen dieses Aerosolgenerators befördert, um das Fluid zu vernebeln.

Besonders vorteilhaft ist es, wenn zur Erzeugung des Aerosolstroms mehrfachdestilliertes Wasser verwendet wird. Hierfür eignet sich beispielsweise zweifachdestilliertes Wasser (Bidestillat) oder dreifachdestilliertes Wasser (Tridestillat). Damit wird eine Reinheit sichergestellt, die den ozonhaltigen Luftstrom nicht beeinträchtigt und damit eine weitere signifikante und reproduzierbare Verbesserung der Keimreduktion sicherzustellen.

Der Aerosolgenerator kann eine Kühleinheit haben und eingerichtet sein, das zur Erzeugung des Aerosolstroms genutzte Fluid, insbesondere Bidestillat oder Tridestillat, auf eine Temperatur von mindestens 5°C unter der Umgebungstemperatur abzukühlen.

Auf diese Weise wird sichergestellt, dass eine Kondensatbildung vermieden wird. Durch die Bildung von Kondensat verringert sich die Wirksamkeit der Destillationsvorrichtung zur Keimreduktion.

Die Kühleinheit ist bevorzugt zur Kühlung des Fluides für die Vernebelung auf eine Temperatur im Bereich von 5°C bis 12°C und bevorzugt in einem Bereich von 8°C bis 10°C eingerichtet. In diesem Temperaturbereich sind die Bedingungen zur Vernebelung von Aerosol und die Zusammenwirkung mit dem ozonhaltigen Luftstrom derart optimal, dass sich die im Aerosolstrom und dem ozonhaltigen Luftstrom zusammenwirkenden Energieverhältnisse nicht gegenseitig beeinträchtigen.

Dies kann insbesondere erreicht werden, wenn die Differenztemperatur zwischen der in der Destillationsvorrichtung vorherrschenden Umgebungstemperatur und der in den Aerosolgenerator eingeleiteten Wassertemperatur mehr als 10°C beträgt.

Die Desinfektionsvorrichtung kann einen Anschlussstutzen zur Aufnahme und zum Anschluss eines Fluidbehälters haben. Der Anschlussstutzen mündet in eine Vernebelungskammer des Aerosolgenerators und ist in Richtung der Schwerkraft gesehen in einer Ebene oberhalb von der Vernebelungskammer angeordnet. Dabei kann der Fluidbehälter direkt über der Vernebelungskammer und besonders vorteilhaft direkt über einem in der Vernebelungskammer angeordneten Verneblers angeordnet sein, so dass das Fluid bei der Entnahme direkt vom Fluidbehälter in die Vernebelungskammer bzw. auf den Vernebler tropft. Der Fluidbehälter kann aber auch in horizontaler Richtung gesehen versetzt zur Vernebelungskammer bzw. einem darin optional eingebauten Vernebler angeordnet sein.

Der Anschlussstutzen kann ein Auslassventil haben, das durch den Differenzdruck zwischen der auf das Auslassventil durch seine Gewichtskraft und der auf das Auslassventil wirkenden Kraft des Fluides sowie des durch den Luftdruck in der Vernebelungskammer auf das Auslassventil wirkenden Gegenkraft verschiebbar im Anschlussstutzen gelagert ist.

Bei dieser Ausführungsform gelingt es durch Erhöhung des Luftdrucks in der Vernebelungskammer während des Desinfektionsprozesses das Auslassventil temporär zu öffnen, um Fluid in die Vernebelungskammer einzuleiten. Durch die vorherrschenden Druckverhältnisse schließt sich dann das Auslassventil automatisch, wenn eine hinreichende Menge von Fluid in die Vernebelungskammer eingeströmt ist. Damit kann auf einfache und zuverlässige Weise nur durch die ohnehin im Betrieb erforderliche Steuerung der Lufteinströmung für den Aerosolstrom Fluid eingeleitet werden. Ein separates, elektronisch angesteuertes Ventil ist hierfür nicht erforderlich. Die Wasserdosierung in die Vernebelungskammer ist damit selbstregulierend.

**In** dem Gehäuse der Desinfektionsvorrichtung kann ein Luftkompressor angeordnet sein, der mit seinem Ausgang mit dem Eingang des Ozongenerators verbunden ist. **In** einer bevorzugten Ausführungsform ist der Luftkompressor zur Ansaugung von Luft aus dem Innenraum des Gehäuses ohne eine spezifisch für den Luftkompressor vorgesehene und in die Umgebung des Gehäuses führende Ansaugöffnung eingerichtet.

Dadurch wird erreicht, dass der in den Plasmagenerator geleitete Luftstrom durch den Betrieb der Desinfektionsvorrichtung angewärmt und durch die Umgebung wenig beeinträchtigt wird. Die in dem Innenraum der Desinfektionsvorrichtung vorherrschende relative Luftfeuchtigkeit ist dabei im Vergleich zur Außenluft besser zur Erzeugung des ozonhaltigen Luftstroms geeignet.

Vorteilhaft ist es, wenn die Desinfektionsvorrichtung einen zusätzlichen Lufttrockner hat, der die zur Trocknung der in den Ozongenerator eingeleiteten Luft auf eine relative Luftfeuchtigkeit von weniger als 50 % und bevorzugt weniger als 30 % eingerichtet ist. Dieser Lufttrockner kann Teil des Plasmagenerators sein. Er kann beispielsweise aus einem Heizelement gebildet werden.

Die Desinfektionsvorrichtung kann einen Luftfilter haben, der in dem Luftstrom vor dem Aerosolgenerator und/oder vor dem Plasmagenerator angeordnet ist. Hierzu eignen sich Taschenfilter, Staubfilter und Trocknungsfilter, wie insbesondere silicagelhaltige Filter oder eine Kombination davon. Mit einem solchen Filter wird die angesaugte Luft nicht nur gereinigt, sondern kann auch zusätzlich getrocknet werden. Ein solcher Luftfilter kann somit auch gleichzeitig als Lufttrockner dienen.

Der Aerosolstrom und der ozonhaltige Luftstrom können in einem Winkel im Bereich von 30 Grad bis 60 Grad zueinander in die Mischkammer eingeleitet werden. Dies hat den Vorteil, dass bei einem solchen Einströmwinkel der Aerosolstrom und der ozonhaltige Luftstrom verwirbeln und damit gut miteinander vermischt werden.

Die Mischkammer kann hierzu beispielsweise einen durch zylinderförmige oder kugelförmige Wandungen begrenzten Raum haben. Durch diese im Schnitt kreisförmige Gestaltung wird die Verwirbelung weiter verbessert.

Die Mischkammer und die Desinfektionskammer, sowie die Verbindungsleitungen zwischen den Kammern sollten aus nichtleitendem Kunststoffmaterial ausgebildet sein. Dies hat den Vorteil, dass mit einem solchen Kunststoffmaterial, wie beispielsweise ABS-Kunststoff, die elektrostatische Entladung insbesondere des ozonhaltigen Luftstroms und des mit dem Aerosolstrom vermischten ozonhaltigen Luftstroms reduziert wird. Damit wird die Wirksamkeit des desinfizierenden aerosol- und ozonhaltigen Luftstroms verbessert.

Die Eindüsungsöffnungen der Desinfektionskammer können in mindestens einem Rohrstück eingebracht sein, das elektrisch isoliert an den die Desinfektionskammer bildenden Wandabschnitten angeordnet ist. Hierbei kann es sich um von den Wandabschnitten separat hergestellten und an diesen angebrachten Rohrstücken handeln. Vorteilhaft ist es, wenn diese Rohrstücke aus einem eloxierten Aluminiumrohr gebildet sind. Denkbar ist aber auch ein Kunststoffrohr.

Die Erfindung wird nachfolgend anhand der beigefügten Zeichnungen näher erläutert. Es zeigen:
- Figur 1 -: Funktionsskizze der Desinfektionsvorrichtung;
- Figur 2 -: perspektivische Schnittansicht einer Desinfektionsvorrichtung gemäß der Funktionsskizze nach Figur 1 mit Mischkammer in einer nicht-erfindungsgemäßen Form;
- Figur 3 -: perspektivische Schnittansicht der Desinfektionsvorrichtung aus Figur 2 mit Teilschnitt im Bereich des Wassereinlasses;
- Figur 4 -: Explosionsansicht der Desinfektionsvorrichtung aus Figuren 2 und 3 mit Blick auf die Unterseite mit der dort angeordneten Mischkammer;
- Figur 5 -: Perspektivische Ansicht der geöffneten Desinfektionsvorrichtung aus Figuren 2 bis 4 ohne umgebendes Außengehäuse mit Blick in die Vernebelungskammer.

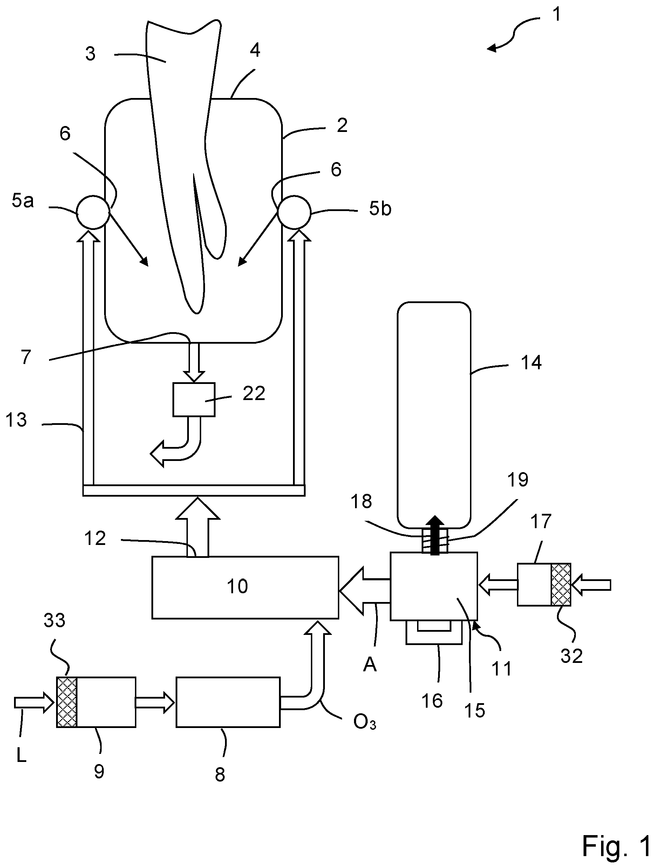

Figur 1 zeigt ein Funktionsdiagramm einer Desinfektionsvorrichtung 1. Diese hat eine Desinfektionskammer 2, die in dem dargestellten Ausführungsbeispiel zum Einführen eines Objektes 3 in Form einer Hand ausgebildet ist, um die Handoberfläche und insbesondere die Finger und Fingerkuppen zu desinfizieren.

Hierzu hat die Desinfektionskammer 2 an der Oberseite mindestens eine Aufnahmeöffnung 4. Diese Aufnahmeöffnung 4 kann auch während des Betriebs geöffnet sein und als Ausschnitt in der Desinfektionskammer 2 ausgebildet werden. Denkbar ist aber auch, dass die Aufnahmeöffnung 4 durch einen flexiblen Vorhang, einen Deckel, einen Luftvorhang oder ähnliches mindestens teilweise während der Desinfektion des in die Desinfektionskammer 2 eingeführten Objektes 3 geschlossen wird.

An den beiden gegenüberliegenden Seitenwänden der Desinfektionskammer 2 sind Eindüsungsrohre 5a, 5b mit Eindüsungsöffnungen 6 angeordnet. Diese Eindüsungsöffnungen 6 sind in den Innenraum der Desinfektionskammer 2 auf die Sollposition der zu desinfizierenden Oberfläche gerichtet.

Die Eindüsungsöffnungen 6 sind vorzugsweise in einem Winkel von etwa 40 Grad bis 70 Grad zur Waagerechten und bevorzugt in einem Winkel von etwa 60 Grad ± 5 Grad zur Waagerechten in den Innenraum der Desinfektionskammer 2 schräg nach unten zur Aufnahmeöffnung 4 ausgerichtet. Damit wird ein direktes Ausströmen des desinfizierenden Aerosolgemischs aus der Öffnung erst dann möglich, wenn der Desinfektionsraum vollständig gefüllt ist. Bei einer optimalen Volumenabstimmung wird so das Austreten weitgehen verhindert.

Der bei der Desinfektion eingeströmte überschüssige ozonhaltige Aerosolnebel kann dann nach oben herausströmen. Bei der Einströmung tritt eine Verdichtung und Verwirbelung im unteren Bereich der Desinfektionskammer 2 auf, die zu einem optimalen Wirkstrom auf die zu desinfizierenden Flächen führt.

Für die Desinfektion von zwei Händen gleichzeitig, die nebeneinander in die Desinfektionskammer 2 eingeführt werden, sollte das Volumen der Desinfektionskammer etwa 2,5 Liter ± 20 % betragen.

Am Boden der Desinfektionskammer 2 befindet sich eine Auslassöffnung 7, über die nach Abschluss des Desinfektionsvorgangs mit Hilfe eines Lüfters 22 oder einer Absaugpumpe die in der Desinfektionskammer 2 verbliebene aerosol- und ozonhaltige Luft über ein Aktivkohlefilter in die Umgebung abgezogen wird. Dabei kann auch sich etwaig ansammelndes Kondensat abgeführt werden.

Die Desinfektion erfolgt mit einem Gemisch aus einem ionisierten Aerosol A zusammen mit ozonhaltiger Luft O₃.

Die ozonhaltige Luft O₃ (auch Plasmagasstrom genannt) wird mit einem Ozongenerator 8 erzeugt, dem über einen Luftkompressor 9 Luft zugeführt wird. Diese zugeführte Luft wird im Ozongenerator 8 einem Hochspannungsfeld ausgesetzt und dort ionisiert. Der Luftkompressor 9 zieht diese Luft aus der Umgebung der Desinfektionsvorrichtung 1 und bevorzugt aus dem Innenraum innerhalb des Außengehäuses der Desinfektionsvorrichtung 1 an. Die sich im Innenraum der Desinfektionsvorrichtung 1 befindliche Luft ist dann bereits durch den Betrieb der Desinfektionsvorrichtung 1 vorgewärmt und in der Regel trockener, als die Umgebungsluft.

In dem Luftkompressor 9 oder mit einer separaten Einrichtung, die vor dem Ozongenerator 8 geschaltet ist, kann die Luft weiter getrocknet werden. Bevorzugt ist ein Betrieb mit einer relativen Luftfeuchtigkeit im Bereich von 20 %. Die relative Luftfeuchtigkeit sollte nach Möglichkeit mindestens kleiner als 50 % sein. Damit lässt sich eine hochwirksame Ozongewinnung aus der Umgebungsluft L sicherstellen.

Das so erzeugte Ozon O₃ wird einer Mischkammer 10 zugeführt.

Weiterhin ist ein Aerosolgenerator 11 zur Erzeugung eines Aerosolstroms A vorhanden, der ebenfalls in die Mischkammer 10 eingeführt wird. Der ozonhaltige Luftstrom O₃ und der Aerosolstrom A werden hierbei in einem Winkel zueinander in die Mischkammer eingeblasen, der im Bereich von 30 Grad bis 60 Grad zueinander liegt. Damit wird erreicht, dass der ozonhaltige Luftstrom O₃ und der Aerosolstrom A miteinander verwirbeln und vermischt werden.

Der Aerosolstrom A sollte der Hauptstrom sein, der sich bereits in der Mischkammer befindet, bevor ihm ein ozonhaltiger Luftstrom O₃ zugeführt wird. Damit wird nicht der ozonhaltige Luftstrom O3 durch die Aerosole angefeuchtet, sondern die Aerosole A werden durch das Ozon ionisiert und erhalten eine desinfizierende Wirkung.

Die Vermischung erfolgt in einer Mischstrecke zwischen dem Einlass des ozonhaltigen Luftstroms O₃ und des Aerosolstroms A bis zu einem Auslass 12 in der Mischkammer 10. Dieser Auslass 12 befindet sich vorzugsweise am Ende der Mischkammer 10. Der Auslass 12 kann an der gleichen Seite sein, wie der Einlass für den ozonhaltigen Luftstrom O₃, oder aber wie skizziert auf der in Einlassrichtung des ozonhaltigen Luftstroms O₃ gesehen gegenüberliegenden Seite. Der Auslass 12 kann aber auch am Ende der Mischkammer 10 quer zur Einströmungsrichtung des ozonhaltigen Luftstroms O₃ und des Aerosolstroms A ausgerichtet sein.

Der Auslass 12 ist über elektrisch isolierte Rohrleitungen 13 mit den Eindüsungsrohren 5a, 5b verbunden. Diese Rohrleitungen 13 sind vorzugsweise aus einem Kunststoffmaterial ausgeführt.

Die Eindüsungsrohre 5a, 5b können ebenso aus Kunststoffmaterial gebildet sein. Vorteilhaft ist jedoch, wenn diese aus einem eloxierten Aluminiumrohr ausgebildet sind, in das sich die Eindüsungsöffnungen 6 in einfacher Weise als Bohrungen einbringen lassen.

Zur Erzeugung des Aerosolstroms A kann ein Fluidbehälter 14 (Wasserbehälter) senkrecht oberhalb einer Vernebelungskammer 15 angeordnet sein. Am Boden oder seitlich zur Vernebelungskammer 15 befindet sich in dem dargestellten Ausführungsbeispiel ein Piezoaktuator 16, der an eine Ansteuerungseinheit angeschlossen ist, mit der der Piezoaktuator 16 angeregt wird. Dieser wird vorzugsweise mit einer Ultraschall-Frequenz so angeregt, dass mit dem Piezoaktuator 16 in Verbindung tretende Wassertröpfchen oder Wasseransammlungen zu Schwingungen angeregt werden und dabei vernebelt werden. Diese durch Ultraschallanregung entstehenden Aerosole steigen dann in der Vernebelungskammer 15 aus und werden mit Hilfe eines in die Vernebelungskammer 15 wirkenden Gebläses 17 als Aerosolstrom A in die Mischkammer 10 geführt.

Alternativ zu der Vernebelung mit einem Piezoaktuator 16 sind auch andere Techniken zur Erzeugung von Aerosol aus Wasser geeignet, wie z. B. eine Verdüsung unter Wasserdruck.

Die Einleitung von Wasser aus dem Fluidbehälter 14 zu dem Aerosolgenerator 11 kann mit Hilfe eines Auslassventils 18 erfolgen, das sich angesteuert durch den mit dem Gebläse 17 erzeugten Luftdruck anhebt und den Weg für das in dem Fluidbehälter 14 befindliche Wasser in die Vernebelungskammer 15 zum Piezoaktuator 16 freigibt.

Wenn nun der Betrieb beendet und der Lüfter 17 ausgeschaltet wird, dann schließt sich das Auslassventil 18 automatisch. Es wird auch bereits dann geschlossen, wenn sich die Druckverhältnisse in der Vernebelungskammer 15 im Zuge des bereits erzeugten Aerosolstroms A so verändern, dass das Auslassventil 18 durch den in der Vernebelungskammer 15 herrschenden Luftdruck nicht mehr angehoben wird.

Damit wird ein selbstregulierendes System erreicht.

Für die Erzeugung des Aerosolstroms A eignet sich möglichst reines Wasser, bevorzugt mehrfachdestilliertes Wasser wie Bidestillat oder Tridestillat, d. h. nachgereinigtes Wasser. Fremdstoffe in dem zur Erzeugung des Aerosolstroms A genutzten Wassers können zu andersartigen, unvorhersehbaren Reaktionen führen. So kann die Reduzierung der Keimzahl wesentlich verbessert werden, wenn statt einfachem destilliertem Wasser Bidestillat genutzt wird. Dies führt zu einer Erhöhung der Log-Stufe um mindestens 0,5.

Vorteilhaft ist es, wenn an dem Fluidbehälter 14 oder im Übergang des Fluidbehälters 14 zum Aerosolgenerator 11 eine Kühleinheit 19 angeordnet ist, um das Wasser kurz vor der Vernebelung auf eine mindestens 5°C unter der Umgebungstemperatur liegende Temperatur abzukühlen. Durch diese Differenztemperatur wird die Gefahr der nachteiligen Bildung von Kondensat reduziert. Bevorzugt ist eine Kühlung des in die Vernebelungskammer 15 eingeführten Wassers auf etwa 8°C bis 10°C.

Für die angesaugte Luft kann zudem ein Luftfilter 32 im Luftstrom vor dem Aerosolgenerator 11 und gegebenenfalls ein Kombinationsfilterelement 33 (Trocknungs- und Staubfilter) auch in den Luftstrom vor dem Ozongenerator 8 vorgesehen sein. Damit wird sichergestellt, dass die angesaugte Luft möglichst wenige Fremdstoffe enthält, welche den Ionisierungseffekt bei der Bildung der ionisierten Aerosole und des Ozons beeinträchtigen könnten. Hier sind Staubfilter und Trockenmaterial, wie beispielsweise Silicagel vorteilhaft, die auch kombiniert als Taschenfilter erhältlich sind. Der Einsatz von Trockenmaterial ist insbesondere zur Filterung des in den Ozongenerator 8 eingeführten Luftstroms vorteilhaft, um so die relative Luftfeuchtigkeit der zugeführten Luft auf einen Bereich von unter 50 % und bevorzugt auf etwa 20 % zu bringen.

Die Regeneration eines solchen Filters gelingt entweder in einer Rückspülphase nach Abschluss eines Desinfektionsprozesses, bei der der mindestens eine Filter getrocknet wird. Da die Desinfektionsvorrichtung 1 in der Regel nicht im Dauerbetrieb genutzt wird, können die Filter aber auch in den Pausen selbsttätig regenerieren.

Der in der Umgebungsluft L vorhandene Sauerstoff wird in dem Ozongenerator 8 zu einem Teil in Ozon O₃, d. h. in Plasmaluft umgewandelt. In der Regel erfolgt die Umwandlung von Sauerstoff etwa zu 20 % bis 25 % in Ozon. Dieser ozonhaltige Luftstrom O₃ ist sehr reaktiv und instabil. Daher ist wie in Figur 1 skizziert eine räumliche Trennung zwischen dem Ozongenerator 8 und dem Aerosolgenerator 11 vorhanden, die beide über Zuleitungen an die gemeinsame Mischkammer 10 angekoppelt sind.

Diese Mischkammer 10 hat in einem Vergleich zu den Zuleitungen größeres Volumen, sodass sich der ozonhaltige Luftstrom O₃ und der Aerosolstrom A in der Mischkammer 10 wieder ausdehnen und verwirbeln können.

Die Mischkammer 10 ist damit kein Teilstück der an den Ozongenerator 8 angeschlossenen Rohrleitung oder der an den Aerosolgenerator 11 angeschlossenen Rohrleitung, sondern eine eigenständige Kammer mit Zu- und Ableitungen sowie einem im Vergleich zu den Querschnitten der Zu- und Ableitungen größeren Querschnitt.

Durch die Vermischung des Aerosolstroms A mit dem ozonhaltigen Luftstrom O₃ in der Mischkammer 10 werden aus den Aerosolen Hydroxylradikale (OH, HOH etc.) gebildet, deren freien Radikale ein Wirkverstärker für das in der ozonhaltigen Luft vorhandene desinfizierende Ozon O₃ bilden.

Durch die Reaktionsstrecke in der Mischkammer 10 wird ein stabiler Prozess mit reproduzierbaren Bedingungen sichergestellt. Da das Ozon stark instabil ist und nicht vorhersehbare Reaktionen hat, um das überschüssige O-Molekül loszuwerden, ist diese kontrollierte Vermischung in der separaten Mischkammer 10 wesentlich, um einen noch hinreichend lang stabilen desinfizierenden Aerosol-Ozon-Luftstrom in der Desinfektionskammer 2 sicherzustellen.

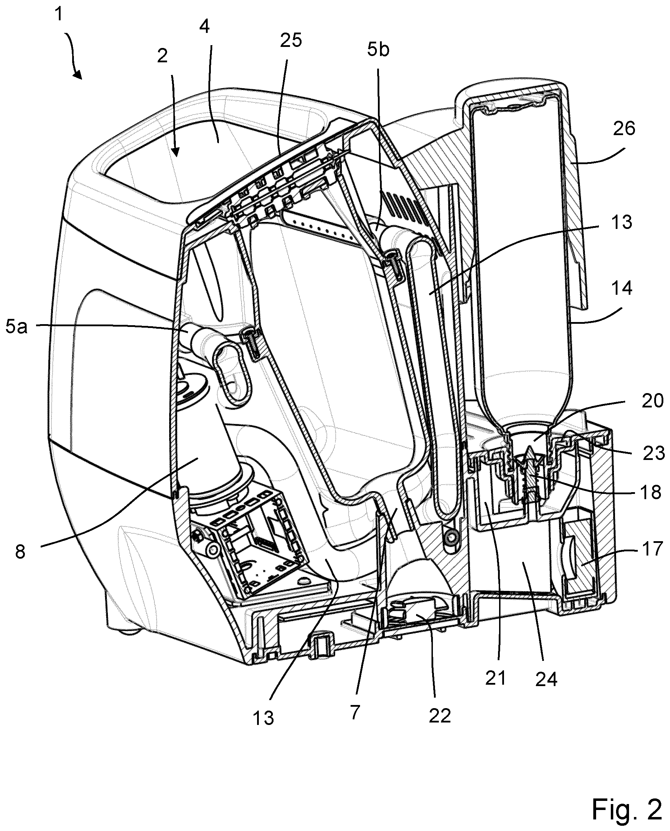

Figur 2 zeigt eine Seiten-Schnittdarstellung einer Desinfektionsvorrichtung 1 gemäß dem Funktionsprinzip aus Figur 1 mit einer Mischkammer 10, die in einer nicht-erfindungsgemäßer Form dargestellt ist. Deutlich wird, dass der Fluidbehälter 14 senkrecht mit seinem Auslass 20 nach unten direkt oberhalb einer Tropfkammer 21 angeordnet ist. In diese Tropfkammer 21 ragt ein Anschlussstutzen 23 hinein, in dem der Fluidbehälter 14 aufgenommen ist. Die Tropfkammer 21 mündet in die in diesem Ausführungsbeispiel seitlich versetzt hierzu angeordnete Vernebelungskammer 15 (in dem Schnitt nicht sichtbar). Am Boden der Vernebelungskammer 15 befindet sich der Piezoaktor 16 (nicht sichtbar) des Aerosolgenerators 11. Das Gebläse 17 ist unterhalb der Tropfkammer 21 angeordnet. Das Gebläse 17 ist so ausgerichtet, dass es durch eine Öffnung an der Rückseite und/oder aus der Unterseite die Luft aus der Umgebung ansaugt und mit einem Luftkanal in die Vernebelungskammer 15 leitet.

Der Aerosolstrom A wird dann von der Vernebelungskammer 15 über einen Kanal in die neben der Vernebelungskammer 15 angeordnete Mischkammer 10 geführt. Wenn die Mischkammer 10 optional unmittelbar neben der Vernebelungskammer 15 liegt hat dies den Vorteil, dass die Strecke des Aerosols in die Mischkammer 10 vorteilhaft verkürzt und die Kondensationsgefahr reduziert wird.

Erkennbar sind auch die Rohrleitungen 13, die zu den beidseits an den Seitenwänden der Desinfektionskammer 2 angeordneten Eindüsungsrohre 5a, 5b führen. Weiterhin ist die Auslassöffnung 7 am Boden der Desinfektionskammer 2 mit dem darunter angeordneten Lüfter 22 erkennbar. Der Ausgang des Lüfters 22 führt am Boden der Desinfektionsvorrichtung 1 in die Umgebung.

Deutlich wird weiterhin, dass der Fluidbehälter 14 im Bereich unterhalb der Auslassöffnung 20 in den Anschlussstutzen 23 eingeschraubt ist. Dieser hat einen Auslassventil 18, dessen kegelförmiges oberes Ende in den Innenraum des Fluidbehälters 14 weist. Dieses Auslassventil 18 ist in senkrechter Richtung linear verschiebbar und wird durch einen in dem neben dem Gebläse 17 liegenden Gebläseraum 24 bzw. in der Vernebelungskammer 15 vorherrschenden Luftdruck angehoben, wenn dieser die Gewichtskraft des Auslassventils 18 und die durch das Fluid in dem Fluidbehälter 14 einwirkende Kraft übersteigt.

Erkennbar ist weiterhin, dass der Anschlussstutzen 23 an seinem Umfang eine Kühleinheit 19 aufweist, mit der das in die Vernebelungskammer 15 eingebrachte Wasser abgekühlt werden kann.

Deutlich wird weiterhin, dass die Desinfektionskammer 2 an ihrer Oberseite zwei durch einen Steg 25 getrennte Aufnahmeöffnungen 4 (nur die linke Aufnahmeöffnung 4 ist durch die Schnittdarstellung dargestellt) aufweist. Diese Aufnahmeöffnung 4 ist vollkommen offen und nicht abschließbar, sodass ein bei der Desinfektion entstehender Luftstrom nach oben ausweichen kann.

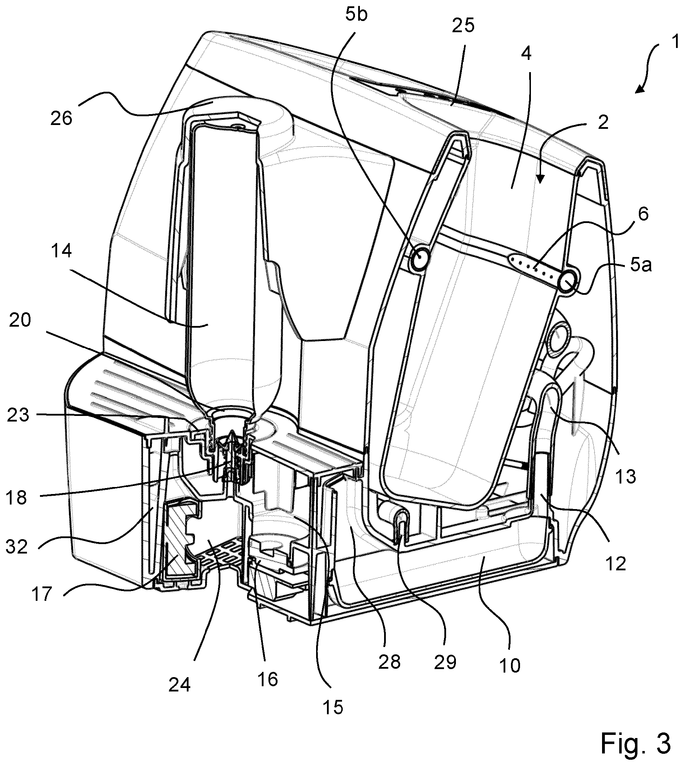

Figur 3 zeigt eine perspektivische Rückseitenansicht der Desinfektionsvorrichtung 1 mit einem durch Teilschnitt freigelegten Blick auf den Fluidbehälter 14 und den Anschlussstutzen 23 mit dem Auslassventil 18.

Deutlich wird, dass das Auslassventil 18 in ein Rohrstück eingesetzt ist und im oberen Bereich eine Manschette hat, welche den Anschlussstutzen 20 abdeckt, wenn das Auslassventil 18 in Richtung der Schwerkraft nach unten verlagert ist. Durch einen sich im Gebläseraum 24 bzw. dem Vernebelungsraum 15 bildenden Luftdruck, der die Gewichtskraft des Auslassventils 2184 sowie die auf das Auslassventil 18 wirkende Kraft des im Fluidbehälter 14 befindlichen Fluids übersteigt, wird das Auslassventil 18 angehoben und gibt eine Auslassöffnung frei, über die Fluid aus dem Fluidbehälter 14 in die Tropfkammer 21 und von dort in Vernebelungskammer 15 einfließen kann. Die Tropfkammer 21 ist am Boden in einem Teilumfang zur unmittelbar angrenzenden Vernebelungskammer 15 hin offen und in Richtung der Schwerkraft gesehen mit seinem Boden oberhalb des Bodens der Vernebelungskammer 15 angeordnet. Dadurch fließt das Fluid aus der Tropfkammer 21 in die Vernebelungskammer 15 hinein und gelangt auf den am Boden der Vernebelungskammer 15 angeordneten Piezoaktuator 16, um durch Ultraschallanregung des Piezoaktuators 16 in Aerosole vernebelt zu werden.

Die durch das im Fluidbehälter 14 befindliche Wasser auf das Auslassventil 18 wirkende Kraft wird dadurch reduziert, dass das in den Fluidbehälter 14 hineinragende Ende konisch zulaufend ist. Es kann wie dargestellt kegelförmig oder auch parabolförmig oder ähnliches sein.

Deutlich wird weiterhin, dass der Fluidbehälter 14 durch einen Deckel 26 abgedeckt und gehalten wird, der auf den Fluidbehälter 14 (beispielsweise eine zylinderförmige Flasche) aufgesteckt und mit dem Gehäuse der Desinfektionsvorrichtung 1 formschlüssig gehalten wird. Der Deckel 26 kann mit dem Gehäuse der Desinfektionsvorrichtung verriegelt oder mittels geeigneter Konturen mittels eines Schlosses gesichert werden.

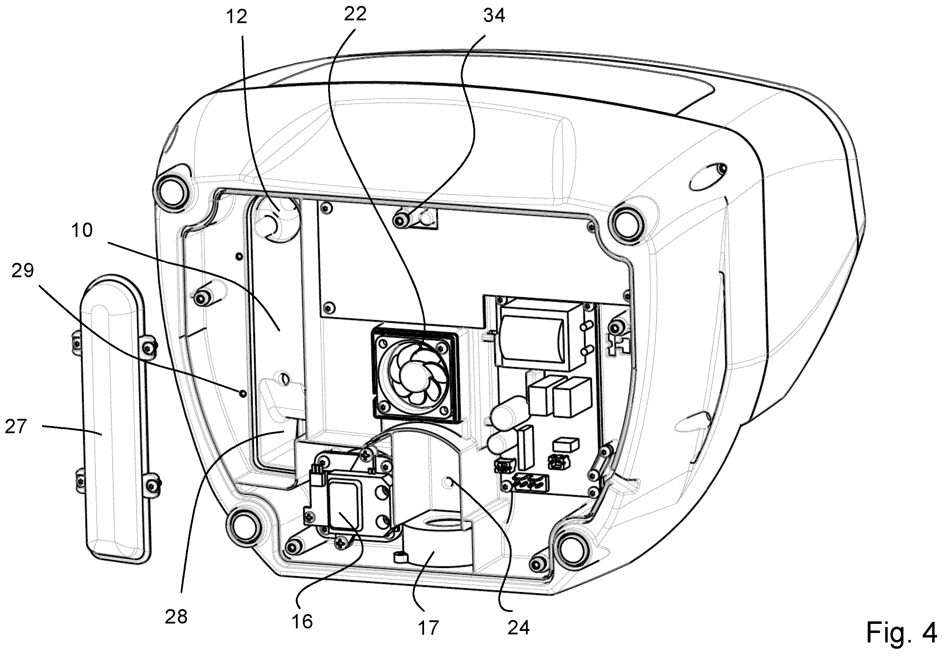

Figur 4 zeigt eine Explosionsansicht der Desinfektionsvorrichtung 1 mit Blick auf den Boden und der dort angeordneten Mischkammer 10, die in einer nicht-erfindungsgemäßen Form dargestellt ist. Die Mischkammer 10 ist durch den in der Darstellung abgenommenen Mischkammerdeckel 27 geöffnet.

Erkennbar ist, dass die Mischkammer 10 eine wesentlich größere Länge, als Breite und Höhe hat. Sie ist in der nicht-erfindungsgemäßen Darstellung in etwa rechteckförmig mit abgerundeten Ecken ausgebildet. In der erfindungsgemäßen Ausführungsform ist die Mischkammer 10 ein Paraboloid.

In der Nähe zu der Vernebelungskammer 15 an der Rückseite ist in einer Bodenfläche der Mischkammer 10 ein Einlass 28 für den Aerosolstrom A vorhanden, der mit einer gekrümmten Fläche in die Bodenebene der Mischkammer 10 übergeht. Dieser Einlass hat einen relativ großen Querschnitt und erstreckt sich in dem Ausführungsbeispiel über die gesamte Breite der Mischkammer 10.

Neben diesem Einlass 28, d. h. relativ nahe hierzu (benachbart), befindet sich ein weiterer Einlass 29 für den ozonhaltigen Luftstrom O₃. Dieser Einlass 29 ist ebenso in den Boden der Mischkammer 10 eingebracht, steht aber durch die vorgelagerte gekrümmte Fläche des Einlasses 28 für den Aerosolstrom A in einem Winkel zu der im Bereich des Einlasses 29 für den ozonhaltigen Luftstrom O₃ vorherrschenden Hauptströmungsrichtung des Aerosolstroms A. Damit wird der ozonhaltige Luftstrom O3, der u.a. durch den geringeren Querschnitt des Einlasses 29 einen geringeren Volumenstrom hat, als der Volumenstrom des durch den deutlich größeren Querschnitt des Einlasses 28 strömenden Aerosolstroms A.

Auf der gegenüberliegenden Seite am Ende in Längserstreckungsrichtung der Mischkammer 10 gesehen befindet sich ebenfalls am Boden der Mischkammer 10 der Auslass 12 für das zur Desinfektion genutzte Gemisch aus Aerosolstrom A und ozonhaltigem Luftstrom O₃ (AO₃). An diesen Auslass 12 sind die Rohrleitungen 13 angeschlossen, mit denen der ionisierte Aerosolstrom A getragen und geschützt durch den ozonhaltigen Luftstrom O3 zu den Eindüsungsrohren 5a, 5b und dessen Eindüsungsöffnungen 6 geleitet wird.

Optional sind aber auch andere Formen der Mischkammer 10 denkbar. Dabei sollte durch einen im Winkel von bevorzugt 30 Grad bis 60 Grad zueinander ausgerichteten Einströmwinkel der beiden Einlässe 28, 29 für den Aerosolstrom A und die ozonhaltige Luft O₃ eine Verwirbelung induziert und über eine ausreichende Wirbelstrecke erreicht werden, dass die Aerosole des Aerosolstroms A durch den ozonhaltigen Luftstrom O₃ ionisieren und sich eine Mischung aus desinfizierenden Aerosolen und einem desinfizierenden Ozonstrom (Plasmagasstrom) bildet.

In der erfindungsgemäßen Ausführungsform ist die Mischkammer 10 ein Paraboloid. In nicht-erfindungsgemäßen Ausführungsformen ist denkbar, dass die Mischkammer 10 beispielsweise eine Kugel, ein Zylinder oder ähnliches ist.

Die Luftzufuhr aus der Atmosphäre kann wie skizziert über ein von dem unteren Geräteraum in den Innenraum des Gerätes führenden Lufteinlassstutzen 34 erfolgen.

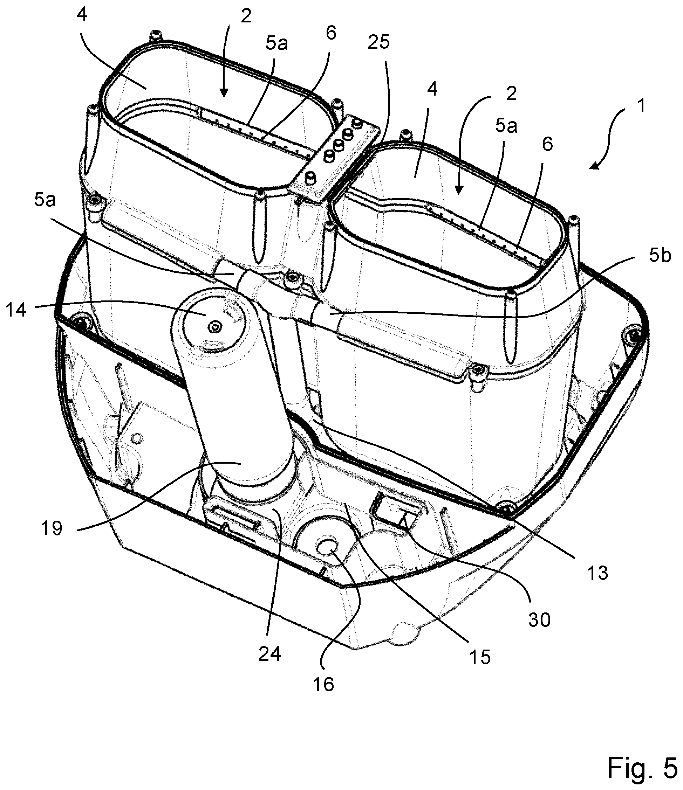

Figur 5 zeigt eine perspektivische Ansicht der geöffneten Desinfektionsvorrichtung 1 aus Figuren 2 bis 4 ohne umgebendes Außengehäuse mit Blick auf den Fluidbehälter 14 und in die Vernebelungskammer 15. Erkennbar ist, dass die Tropfkammer 24 durch eine seitliche Öffnung in die Vernebelungskammer 15 einmündet. Dabei ist der Boden der Tropfkammer 24 in diesem Mündungsübergang zum Boden der Vernebelungskammer 15 hin gekrümmt. Am Boden der Vernebelungskammer 15 befindet sich der Piezoaktuator 16. Die Anordnung ist derart, dass das Wasser aus der Tropfkammer 24 auf den Piezoaktuator 16 fließt.

Die Vernebelungskammer 15 hat an der Seite, die der Tropfkammer 24 gegenüberliegt, eine Trennwand, die den Aerosolkanal 30 bildet, der an seinem anderen Ende den Einlass 28 in die Mischkammer 10 aufweist. Der Aerosolkanals 30 mündet durch einen Spalt oberhalb der Trennwand in die Vernebelungskammer 15.

Erkennbar ist auch, dass die Desinfektionskammer 2 durch den Steg 25 in zwei Raumbereiche aufgeteilt ist, um zwei Hände gleichzeitig aufzunehmen. Für jeden Raumbereich ist jeweils ein Abschnitt der Eindüsungsrohre 5a, 5b vorgesehen. Unterhalb des Stegs 25 sind die Raumbereiche nicht getrennt voneinander, sondern bilden ein einheitliches Luftvolumen.

Der Steg 25 kann zur Aufnahme elektronischer Anzeigeelemente genutzt werden, um dem Nutzer den Betriebszustand der Desinfektionsvorrichtung 1 anzuzeigen.

## Patentansprüche

1. Desinfektionsvorrichtung (1) zur Desinfektion von Oberflächen mit einem Ozongenerator (8) zur Erzeugung eines ozonhaltigen Luftstroms (O₃), einem Aerosolgenerator (11) zur Erzeugung eines wässrige Partikel enthaltenen Aerosolstroms (A) und einer Desinfektionskammer (2), die eine Einlassöffnung (4) zur Einführung eines Objektes (3) mit der zu desinfizierenden Oberfläche in die Desinfektionskammer (2) hat, wobei der Ausgang des Aerosolgenerators (11) mit dem Ausgang des Ozongenerators (8) gekoppelt ist und der von dem Ozongenerator (8) erzeugte ozonhaltige Luftstrom (O₃) mit dem Aerosolstrom (A) des Aerosolgenerators (11) gemischt und in die Desinfektionskammer (2) geleitet wird, wobei eine Mischkammer (10) vorgesehen ist und der Aerosolgenerator (11) und der Ozongenerator (8) mit der Mischkammer (10) zum Einleiten des Aerosolstroms (A) und des ozonhaltigen Luftstroms (O₃) in die Mischkammer (10) verbunden sind, und die Desinfektionskammer (2) Eindüsungsöffnungen (6) hat, die mit der Mischkammer (10) verbunden sind, um den mit dem ozonhaltigen Luftstrom (O₃) vermischten Aerosolstrom (A) als Desinfektionsmedium in die Desinfektionskammer (2) zur Desinfektion der Oberfläche eines in die Desinfektionskammer (2) eingeführten Objektes (3) einzuleiten, **dadurch gekennzeichnet, dass** die Mischkammer (10) ein Paraboloid ist.

2. Desinfektionsvorrichtung (1) nach Anspruch 1, **dadurch gekennzeichnet, dass** die Mischkammer (10) so eingerichtet ist, dass der ozonhaltige Luftstrom (O₃) in den als Hauptstrom in die Mischkammer (10) eingeströmten Aerosolstrom (A) eingemischt wird,

3. Desinfektionsvorrichtung (1) nach Anspruch 1 oder 2, **dadurch gekennzeichnet, dass** die Mischkammer (10) über Rohrleitungen mit dem Ozongenerator (8) und dem Aerosolgenerator (11) verbunden ist und einen größeren Querschnitt als diese Rohrleitungen aufweist.

4. Desinfektionsvorrichtung (1) nach einem der vorhergehenden Ansprüche, **dadurch gekennzeichnet, dass** der Aerosolgenerator (11) eine Kühleinheit (19) hat und eingerichtet ist, das zur Erzeugung des Aerosolstroms (A) genutzte Fluid, insbesondere mehrfachdestilliertes Wasser, auf eine Temperatur von mindestens 5°C unter der Umgebungstemperatur abzukühlen.

5. Desinfektionsvorrichtung (1) nach Anspruch 4, **dadurch gekennzeichnet, dass** die Kühleinheit (19) zur Kühlung des Fluides für die Vernebelung auf eine Temperatur im Bereich von 5°C bis 12°C und bevorzugt im Bereich von 8°C bis 10°C eingerichtet ist.

6. Desinfektionsvorrichtung (1) nach einem der vorhergehenden Ansprüche, **gekennzeichnet durch** einen Anschlussstutzen (20) zur Aufnahme und Anschluss eines Fluidbehälters (14), wobei der Anschlussstutzen (20) in eine Vernebelungskammer (15) des Aerosolgenerators (11) mündet und in Richtung der Schwerkraft gesehen in einer Ebene oberhalb von der Vernebelungskammer (15) angeordnet ist, wobei der Anschlussstutzen (20) ein Auslassventil (18) hat, das durch den Differenzdruck zwischen der auf das Auslassventil durch eine Gewichtskraft und der auf das Auslassventil (18) wirkenden Kraft des Fluides sowie des durch den Luftdruck in der Vernebelungskammer (15) auf das Auslassventil (18) wirkenden Gegenkraft verschiebbar im Anschlussstutzen (20) gelagert ist.

7. Desinfektionsvorrichtung (1) nach einem der vorhergehenden Ansprüche, **gekennzeichnet durch** einen Anschlussstutzen (20) zur Aufnahme und Anschluss eines Fluidbehälters (14), wobei der Anschlussstutzen (20) über eine Dosierpumpe mit dem Aerosolgenerator (11) verbunden ist.

8. Desinfektionsvorrichtung (1) nach einem der vorhergehenden Ansprüche, **dadurch gekennzeichnet, dass** in einem Gehäuse der Desinfektionsvorrichtung (1) ein Luftkompressor (9) angeordnet ist, der mit seinem Ausgang mit dem Ozongenerator (8) verbunden ist, wobei der Luftkompressor (9) zur Ansaugung von Luft aus dem Innenraum des Gehäuses ohne eine in die Umgebung des Gehäuses führende und spezifisch für den Luftkompressor (9) vorgesehene Ansaugöffnung eingerichtet ist.

9. Desinfektionsvorrichtung (1) nach einem der vorhergehenden Ansprüche, **dadurch gekennzeichnet, dass** die Desinfektionsvorrichtung (1) einen Lufttrockner hat, der zur Trocknung der in den Ozongenerator (8) und/oder den Aerosolgenerator (11) eingeleiteten Luft auf eine relative Luftfeuchte von weniger als 50% und bevorzugt weniger als 30 % eingerichtet ist.

10. Desinfektionsvorrichtung (1) nach einem der vorhergehenden Ansprüche, **gekennzeichnet durch** einen Luftfilter, der im Luftstrom vor dem Ozongenerator (8) und/oder Aerosolgenerator (11) angeordnet ist.

11. Desinfektionsvorrichtung (1) nach einem der vorhergehenden Ansprüche, **dadurch gekennzeichnet, dass** der Aerosolstrom (A) und der ozonhaltige Luftstrom (O₃) in einem Winkel im Bereich von 30 Grad bis 60 Grad zueinander in die Mischkammer (10) eingeleitet werden.

12. Desinfektionsvorrichtung (1) nach einem der vorhergehenden Ansprüche, **dadurch gekennzeichnet, dass** die Mischkammer (10) und die Desinfektionskammer (2) aus nichtleitendem Kunststoffmaterial ausgebildet sind.

13. Desinfektionsvorrichtung (1) nach einem der vorhergehenden Ansprüche, **dadurch gekennzeichnet, dass** die Eindüsungsöffnungen (6) der Desinfektionskammer (2) in mindestens ein Eindüsungsrohr (5a, 5b) eingebracht sind, das elektrisch isoliert an den die Desinfektionskammer (2) ausbildenden Wandabschnitten angeordnet ist.

14. Verwendung von destilliertem Wasser bei einer Desinfektionsvorrichtung (1) nach einem der vorhergehenden Ansprüche, **dadurch gekennzeichnet, dass** der Aerosolgenerator (11) eingerichtet ist, um den Aerosolstrom (A) durch Vernebelung mittels Ultraschall von dem verwendeten destillierten Wasser zu erzeugen.

15. Verwendung von destilliertem Wasser bei einer Desinfektionsvorrichtung (1) nach einem der Ansprüche 1 bis 11, **dadurch gekennzeichnet, dass** der Aerosolgenerator (11) eingerichtet ist, um den Aerosolstrom (A) durch Eindüsen von dem verwendeten destillierten Wasser mittels Düsen zu erzeugen.

16. Verwendung nach Anspruch 12 oder 13, **dadurch gekennzeichnet, dass** zur Erzeugung des Aerosolstroms (A) mehrfachdestilliertes Wasser verwendet wird.

## Claims

1. Disinfection device (1) for disinfecting surfaces, comprising an ozone generator (8) for generating an ozone-containing air stream (O₃), an aerosol generator (11) for generating an aerosol stream (A) containing aqueous particles, and a disinfection chamber (2) having an inlet opening (4) for introducing an object (3) with the surface to be disinfected into the disinfection chamber (2), wherein the outlet of the aerosol generator (11) is coupled to the outlet of the ozone generator (8), and the ozone-containing air stream (O₃) generated by the ozone generator (8) is mixed with the aerosol stream (A) of the aerosol generator (11) and fed into the disinfection chamber (2), wherein a mixing chamber (10) is provided and wherein the aerosol generator (11) and the ozone generator (8) are connected to the mixing chamber (10) for introducing the aerosol stream (A) and the ozone-containing air stream (O₃) are connected to the mixing chamber (10), and the disinfection chamber (2) has injection openings (6) which are connected to the mixing chamber (10) in order to introduce the aerosol stream (A) mixed with the ozone-containing air stream (O₃) as a disinfection medium into the disinfection chamber (2) for disinfecting the surface of an object (3) introduced into the disinfection chamber (2), **characterized in that** the mixing chamber (10) is a paraboloid.

2. Disinfection device (1) according to claim 1, **characterized in that** the mixing chamber (10) is arranged so that the ozone-containing air stream (O₃) is mixed into the aerosol stream (A) flowing into the mixing chamber (10) as the main stream.

3. Disinfection device (1) according to claim 1 or 2, **characterized in that** the mixing chamber (10) is connected to the ozone generator (8) and the aerosol generator (11) via pipes and has a larger cross-section than these pipes.

4. Disinfection device (1) according to one of the preceding claims, **characterized in that** the aerosol generator (11) comprises a cooling unit (19) and is designed to cool the fluid used to generate the aerosol stream (A), in particular multiply distilled water, to a temperature of at least 5°C below the ambient temperature.

5. Disinfection device (1) according to claim 4, **characterized in that** the cooling unit (19) is designed to cool the fluid for nebulization to a temperature in the range of 5°C to 12°C and preferably in the range of 8°C to 10°C.

6. Disinfection device (1) according to one of the preceding claims, **characterized by** a connecting piece (20) for receiving and connecting a fluid container (14), wherein the connecting piece (20) opens into a nebulization chamber (15) of the aerosol generator (11) and is arranged in a plane above the nebulization chamber (15) as seen in the direction of gravity, wherein the connecting piece (20) has an outlet valve (18) which is displaceably mounted in the connecting piece (20) by the differential pressure between the force acting on the outlet valve due to a weight force and the force of the fluid acting on the outlet valve (18) as well as the counterforce acting on the outlet valve (18) due to the air pressure in the nebulization chamber (15).

7. Disinfection device (1) according to one of the preceding claims, **characterized by** a connecting piece (20) for receiving and connecting a fluid container (14), wherein the connecting piece (20) is connected to the aerosol generator (11) via a metering pump.

8. Disinfection device (1) according to one of the preceding claims, **characterized in that** an air compressor (9) is arranged in a housing of the disinfection device (1), which is connected by its output to the ozone generator (8), wherein the air compressor (9) is designed to suck in air from the interior of the housing without an intake opening leading into the surroundings of the housing and provided specifically for the air compressor (9).

9. Disinfection device (1) according to one of the preceding claims, **characterized in that** the disinfection device (1) has an air dryer which is designed to dry the air introduced into the ozone generator (8) and/or the aerosol generator (11) to a relative humidity of less than 50% and preferably less than 30%.

10. Disinfection device (1) according to one of the preceding claims, **characterized by** an air filter arranged in the air stream upstream of the ozone generator (8) and/or aerosol generator (11).

11. Disinfection device (1) according to one of the preceding claims, **characterized in that** the aerosol stream (A) and the ozone-containing air stream (O₃) are introduced into the mixing chamber (10) at an angle in the range of 30 degrees to 60 degrees to one another.

12. Disinfection device (1) according to one of the preceding claims, **characterized in that** the mixing chamber (10) and the disinfection chamber (2) are made of nonconductive plastic material.

13. Disinfection device (1) according to one of the preceding claims, **characterized in that** the injection openings (6) of the disinfection chamber (2) are introduced into at least one injection tube (5a, 5b) which is arranged in an electrically insulated manner on the wall sections forming the disinfection chamber (2).

14. Use of distilled water in a disinfection device (1) according to one of the preceding claims, **characterized in that** the aerosol generator (11) is arranged to generate the aerosol stream (A) by atomization of the used distilled water by means of ultrasound.

15. Use of distilled water in a disinfection device (1) according to one of claims 1 to 11, **characterized in that** the aerosol generator (11) is arranged to generate the aerosol stream (A) by injecting distilled water by means of nozzles.

16. Use according to claim 12 or 13, **characterized in that** multiply distilled water is used to generate the aerosol stream (A).

## Revendications

1. Dispositif de désinfection (1) pour désinfecter des surfaces, comprenant un générateur d'ozone (8) pour générer d'un flux d'air (O₃) contenant de l'ozone, un générateur d'aérosol (11) pour générer un flux d'aérosol (A) contenant des particules aqueuses, et une chambre de désinfection (2) qui présente une ouverture d'entrée (4) pour introduire dans la chambre de désinfection (2) un objet (3) présentant la surface à désinfecter,
dans lequel
la sortie du générateur d'aérosol (11) est couplée à la sortie du générateur d'ozone (8), et le flux d'air (O₃) contenant de l'ozone, généré par le générateur d'ozone (8), est mélangé au flux d'aérosol (A) du générateur d'aérosol (11) et est introduit dans la chambre de désinfection (2),
il est prévu une chambre de mélange (10), et le générateur d'aérosol (11) et le générateur d'ozone (8) sont reliés à la chambre de mélange (10) pour introduire le flux d'aérosol (A) et le flux d'air (O₃) contenant de l'ozone dans la chambre de mélange (10), et la chambre de désinfection (2) présente des ouvertures d'injection (6) qui sont reliées à la chambre de mélange (10) pour introduire dans la chambre de désinfection (2) le flux d'aérosol (A) mélangé au flux d'air (O₃) contenant de l'ozone comme milieu de désinfection pour désinfecter la surface d'un objet (3) introduit dans la chambre de désinfection (2),
**caractérisé en ce que** la chambre de mélange (10) est un paraboloïde.

2. Dispositif de désinfection (1) selon la revendication 1,
**caractérisé en ce que** la chambre de mélange (10) est agencée de telle sorte que le flux d'air (O₃) contenant de l'ozone est mélangé au flux d'aérosol (A) qui s'écoule comme flux principal dans la chambre de mélange (10).

3. Dispositif de désinfection (1) selon la revendication 1 ou 2,
**caractérisé en ce que** la chambre de mélange (10) est reliée au générateur d'ozone (8) et au générateur d'aérosol (11) par des conduits tubulaires et présente une section transversale supérieure à celle de ces conduits tubulaires.

4. Dispositif de désinfection (1) selon l'une des revendications précédentes, **caractérisé en ce que** le générateur d'aérosol (11) comporte une unité de refroidissement (19) et est agencé pour refroidir le fluide, en particulier de l'eau distillée plusieurs fois, utilisé pour générer le flux d'aérosol (A), à une température inférieure d'au moins 5 °C à la température ambiante.

5. Dispositif de désinfection (1) selon la revendication 4,
**caractérisé en ce que** l'unité de refroidissement (19) est agencée pour refroidir le fluide pour la nébulisation à une température dans la plage de 5°C à 12 °C et de préférence dans la plage de 8 °C à 10 °C.

6. Dispositif de désinfection (1) selon l'une des revendications précédentes, **caractérisé par** une tubulure de raccordement (20) destinée à recevoir et à raccorder un réservoir de fluide (14), la tubulure de raccordement (20) débouchant dans une chambre de nébulisation (15) du générateur d'aérosol (11) et étant disposée, vue dans le sens de la gravité, dans un plan situé au-dessus de la chambre de nébulisation (15), la tubulure de raccordement (20) comprenant une vanne de sortie (18) qui est montée dans la tubulure de raccordement (20) de manière à pouvoir se déplacer sous l'effet de la pression différentielle entre la force du fluide agissant sur la vanne de sortie par un poids et la force antagoniste agissant sur la vanne de sortie (18) par la pression de l'air dans la chambre de nébulisation (15).

7. Dispositif de désinfection (1) selon l'une des revendications précédentes, **caractérisé par** une tubulure de raccordement (20) destinée à recevoir et à raccorder un réservoir de fluide (14), la tubulure de raccordement (20) étant reliée au générateur d'aérosol (11) par une pompe de dosage.

8. Dispositif de désinfection (1) selon l'une des revendications précédentes, **caractérisé en ce qu'**un compresseur d'air (9) est disposé dans un boîtier du dispositif de désinfection (1) et est relié par sa sortie au générateur d'ozone (8), le compresseur d'air (9) étant agencé pour aspirer de l'air hors de l'intérieur du boîtier, et ceci sans ouverture d'aspiration débouchant dans l'environnement du boîtier et prévue spécifiquement pour le compresseur d'air (9).

9. Dispositif de désinfection (1) selon l'une des revendications précédentes, **caractérisé en ce que** le dispositif de désinfection (1) comporte un sécheur d'air agencé pour sécher l'air introduit dans le générateur d'ozone (8) et/ou dans le générateur d'aérosol (11) à une humidité relative inférieure à 50 % et de préférence inférieure à 30 %.

10. Dispositif de désinfection (1) selon l'une des revendications précédentes, **caractérisé par** un filtre à air disposé dans le flux d'air en amont du générateur d'ozone (8) et/ou du générateur d'aérosol (11).

11. Dispositif de désinfection (1) selon l'une des revendications précédentes, **caractérisé en ce que** le flux d'aérosol (A) et le flux d'air (O₃) contenant de l'ozone sont introduits dans la chambre de mélange (10) selon un angle compris entre 30 degrés et 60 degrés l'un par rapport à l'autre.

12. Dispositif de désinfection (1) selon l'une des revendications précédentes, **caractérisé en ce que** la chambre de mélange (10) et la chambre de désinfection (2) sont réalisées en matière plastique non conductrice.

13. Dispositif de désinfection (1) selon l'une des revendications précédentes, **caractérisé en ce que** les ouvertures d'injection (6) de la chambre de désinfection (2) sont ménagées dans au moins un tube d'injection (5a, 5b) disposé de manière électriquement isolée sur les portions de paroi formant la chambre de désinfection (2).

14. Utilisation d'eau distillée dans un dispositif de désinfection (1) selon l'une des revendications précédentes,
**caractérisée en ce que** le générateur d'aérosol (11) est agencé pour générer le flux d'aérosol (A) par nébulisation par ultrasons de l'eau distillée utilisée.

15. Utilisation d'eau distillée dans un dispositif de désinfection (1) selon l'une des revendications 1 à 11,
**caractérisée en ce que** le générateur d'aérosol (11) est agencé pour générer le flux d'aérosol (A) par injection de l'eau distillée utilisée au moyen de buses.

16. Utilisation selon la revendication 12 ou 13,
**caractérisée en ce que** de l'eau distillée plusieurs fois est utilisée pour générer le flux d'aérosol (A).
